(19) **Europäisches Patentamt**
**European Patent Office**
**Office européen des brevets**

(11) **EP 3 565 455 B1**

(12) **EUROPEAN PATENT SPECIFICATION**

(45) Date of publication and mention
of the grant of the patent:
**06.03.2024  Bulletin 2024/10**

(21) Application number: **18700530.1**

(22) Date of filing: **04.01.2018**

(51) International Patent Classification (IPC):
**A61B 5/00** *(2006.01)*     **A61B 5/103** *(2006.01)*
**A45D 44/00** *(2006.01)*

(52) Cooperative Patent Classification (CPC):
**A61B 5/0079; A61B 5/103; A61B 5/444;
A61B 5/6803; A61B 5/6898;** A45D 2044/007

(86) International application number:
**PCT/EP2018/050177**

(87) International publication number:
**WO 2018/127529 (12.07.2018 Gazette 2018/28)**

(54) **DEVICE FOR ACQUIRING A VIDEO OF SKIN ALTERATIONS, ASSOCIATED CHARACTERIZATION SYSTEM AND CHARACTERIZATION METHOD**

VORRICHTUNG ZUR ERFASSUNG EINES VIDEOS VON HAUTVERÄNDERUNGEN, ZUGEHÖRIGES CHARAKTERISIERUNGSSYSTEM UND CHARAKTERISIERUNGSVERFAHREN

DISPOSITIF D'ACQUISITION D'UNE VIDÉO D'ALTÉRATIONS DE LA PEAU, SYSTÈME DE CARACTÉRISATION ET PROCÉDÉ DE CARACTÉRISATION ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.01.2017  FR 1750067**

(43) Date of publication of application:
**13.11.2019  Bulletin 2019/46**

(73) Proprietor: **L'OREAL
75008 Paris (FR)**

(72) Inventors:
 • **FLAMENT, Frédéric
94152 Chevilly Larue (FR)**
 • **FRANCOIS, Ghislain
92110 Clichy (FR)**
 • **KNOOK, Eilien
5633 AC Eindhoven (NL)**

 • **ARKESTEIJN, Walter
5633 AC Eindhoven (NL)**
 • **BUIJS, Siedse
5633 AC Eindhoven (NL)**
 • **VAN HALEN, Arnoud
5633 AC Eindhoven (NL)**

(74) Representative: **Lavoix
2, place d'Estienne d'Orves
75441 Paris Cedex 09 (FR)**

(56) References cited:
**FR-A1- 2 930 826     US-A1- 2015 316 771
US-B1- 6 433 760**

 • **LE HOANG ET AL: "Eye Blink Detection for Smart Glasses", 2013 IEEE INTERNATIONAL SYMPOSIUM ON MULTIMEDIA, IEEE, 9 December 2013 (2013-12-09), pages 305-308, XP032570708, DOI: 10.1109/ISM.2013.59**

**Description**

This invention relates to a device for acquiring a video of skin alterations of a user, and particularly wrinkle type skin alterations. The invention also relates to an associated system for characterization of such skin alterations. The invention also relates to a characterization method associated with such a system. The invention also relates to uses of such a system.

Skin alterations are most particularly skin relief, and particularly wrinkles. As a variant, skin alterations consist of micro-relief, eye bags, pores, pimples, moles, scars or any other excrescence or dimple present on the face.

Skin alterations may also be complexion type alterations such as wrinkles or skin discolorations.

More specifically, skin relief may include hair or keratinic fibers projecting from the skin and particularly from the eyelashes of a user.

Cosmetic products with an "anti-wrinkle" effect have been developed in order to make wrinkles less visible. The purpose of such cosmetic products is to make cutaneous relief of the skin uniform. A "cosmetic product" is more generally a product as defined in EC Regulation number 1223/2009 of the European Parliament and the Council of November 30, 2009, relating to cosmetic products.

Other related prior art:

LE HOANG ET AL: "Eye Blink Detection for Smart Glasses",2013 IEEE INTERNATIONAL SYMPOSIUM ON MULTIMEDIA, IEEE, 9 December 2013 (2013-12-09), pages 305-308, XP032570708,DOI: 10.1109/ISM.2013.59 discloses a frame with integrated camera for detecting eye blinking.

FR2930826A1 discloses a frame with integrated direct illumination of the face of a user when wearing.

US6433760B1 discloses a face portable frame with integrated cameras and illumination to track the the eyes of the user.

US2015/316771A1 discloses a head-worn display with multiply folded optics for providing a displayed image overlaid onto a see-through view of a surrounding environment.

There are also cosmetic products specifically for the treatment of other types of skin alterations. For example and particularly, anti-dark circle and anti-discoloration products.

Evaluation methods have been developed in order to evaluate the effects of cosmetic products and to get a better understanding of aging mechanisms.

In particular, methods are known for evaluating wrinkles statically, in other words making use of data acquired at a precise and fixed moment in time. For example such data include images, scans and measurements.

However, data acquired by such static methods do not represent the complete diversity of real conditions under which skin alterations and particularly wrinkles are studied, as they are perceived by users in their environment as a function of their attitudes and uncontrolled and varied illumination conditions.

Furthermore, measurements made represent the state of skin alterations at a fixed instant. However, in everyday life, the perception of said skin alterations, and particularly wrinkles, is dynamic, for example when the user puckers or closes his or her eyes or when he or she changes the orientation of the face in front of a mirror.

Furthermore, regular face movements also contribute to the appearance of the first wrinkles, particularly so-called expression wrinkles. Wrinkles around the contour of the eye, also called crow's feet, are the first to appear, at the age of about 25-30 years. The skin around the contour of the eye is thinner and very mobile due to repeated smiling. Therefore one challenge consists of acquiring information about such movements, particularly for predictive purposes.

Concerning eyelash type alterations, regular movements consisting of blinking the eyes and closing of the eyelids and stresses such as a variation to the humidity or temperature during a day can also have an impact on eyelashes. Therefore another challenge consists of evaluating the effectiveness and resistance of a cosmetic product under real conditions.

Thus, such static methods cannot be used to reconstitute dynamic changes to skin alterations to be studied, and particularly wrinkles.

Methods of dynamically evaluating wrinkles are also known. For example, FR 3 014 675 A describes a method of evaluating clinical signs of the face in movement.

However, methods used in the past can only be used under standard conditions, in other words that are not representative of the real life of users.

Therefore there is a need for a device for acquiring images of skin alterations in real time, dynamically and under everyday conditions.

To achieve this, the purpose of the invention is a device for acquiring a video of skin alterations of a user, the user having a face comprising at least one skin alteration and two eyes, the skin alteration(s) being permanent alterations or alterations that appear during a movement of the user's face, the device comprising:

- a frame is intended (which can also be expressed by a future, that is here, that will be) placed on the user's face in front of the user's eyes, and
- a camera fixed on the frame, the camera comprising a field of vision, the camera being fixed on the frame such that a part of the user's face is directly or indirectly in the field of vision of the camera when the frame is placed on the user's face in front of the user's eyes, said part of the face comprising at least one skin alteration, and an image of said part of the face being intended to be acquired by the camera.

Thus, in providing a camera associated with a frame, the camera will be fixed relative to the user's face so that the variation of the skin alteration can be studied directly during an activity under real conditions.

In particular, such a device can be used to characterize skin alterations such as wrinkles and therefore subjacently the effects of associated cosmetic products, and particularly "anti-wrinkle" products.

Since characterization is made from videos acquired in real time, it can be used to dynamically monitor the development of skin alterations such as wrinkles under everyday conditions. Thus, such a system can help to get a better understanding of occasional variations of wrinkles during a day. Characterization also makes it possible during a day to capture changes to a skin alteration exposed to changing environmental conditions (exposure to the sun, temperature, humidity). To achieve this, the device may include additional sensors capable of measuring such environmental parameters.

Such a system can provide access to images of skin alterations, particularly wrinkles, so that the visibility of skin alterations can be measured at each instant and under real conditions. The data thus acquired can give a better understanding of isolated movements (smile, puckering, expression, language, physical effort) and environmental parameters on the studied skin alteration and how they change during the day and their impact on visibility at the end of the day ("tired look" effect, particularly for wrinkles, dark rings, etc.).

Such a system can also be used to evaluate the benefits of cosmetic products such as "anti-wrinkle" products throughout the life of the product and at key moments. Examples of key moments are just after application of the cosmetic product such as an "anti-wrinkle" cosmetic product or an "anti-wrinkle" foundation or just after a sports session or at a precise moment at which the user feels the need to reapply makeup.

Therefore the acquiring device can be used for the acquisition of images of skin alterations in real time, dynamically and under everyday conditions.

The device according to the invention may comprise one or several of the following characteristics taken in isolation or in any technically possible combination:

- the camera is fixed on the frame such that when the frame is placed on the user's face in front of his or her eyes, the part of the face that is directly or indirectly in the field of vision of the camera is part of a peripheral zone of one of the user's eyes.

Such an arrangement of the camera is particularly suitable for acquiring a video of the user's crow's foot.

- each of the user's eyes comprises an iris, the field of vision of the camera being directly or indirectly centered on a part of the user's face except for the iris when the frame is placed on the user's face in front of the user's eyes.

Therefore such an arrangement of the camera is such that the acquired images are different from the images that would be acquired by an iris tracking (eye tracking) system.

- the device comprises at least one lighting unit fixed to the frame, the lighting unit advantageously comprising a light emitting diode, the lighting unit being capable of lighting at least part of the face directly or indirectly in the field of vision of the camera and that will be acquired by the camera.

Such a light source can illuminate the part of the user's face acquired by the camera and therefore improve the visibility of skin alterations present on such a part of the face.

If necessary, the light source can also make brightness conditions uniform and assure their reproducibility. The light source also enables better image processing of the images after they have been captured. Thus, images of the part of the user's face acquired by the camera have better quality and in particular better luminosity and/or better contrast.

- the camera comprises an objective not oriented directly towards the part of the face to be acquired by the camera, the device also comprising a first optical reflection unit oriented such that at least some of the light waves emitted by the part of the face for which the image is to be acquired are reflected to the camera objective.
- the first optical reflection unit comprises two mirrors positioned such that the video acquired by the camera is a stereoscopic video.

Thus, the first optical reflection unit makes it possible to position the camera remotely, which contributes to making the device compact. In particular, such a first optical reflection unit makes it possible to capture the light path such that the image of the zone to be captured (part of the user's face) is reflected to the camera objective.

- the lighting unit is not oriented directly towards the part of the face intended to be acquired by the camera, the device also comprising a second optical reflection unit oriented such that at least some of the light waves emitted by the lighting unit are reflected to said part of the face, the first optical reflection unit advantageously being coincident with the second optical reflection unit.

The second optical reflection unit makes it possible for the light source to be installed remotely. In particular, such a second optical reflection unit makes it possible to send some of the light waves emitted by the light source to the zone to be captured so as to illuminate said zone to be captured. Preferably, the light source is placed close to the camera objective and the first and the second optical reflection units are coincident. Thus, the device is more compact.

- the frame comprises two circles or half-circles intended to be positioned in front of the user's eyes and temples each connecting a circle or half-circle to one of the user's ears, each temple being intended to rest on one of the user's ears, the camera being fixed to one of the temples.

Such circles or half-circles will be fitted with corrective lenses adapted to the user such that the user does not realize that he or she is wearing a device different from his or her ordinary spectacles. This optimizes discretion of the acquiring device.

The invention also relates to a system for characterization of skin alterations of a user, the user having a face comprising at least one skin alteration and two eyes, the skin alteration(s) being permanent alterations or alterations that appear during a movement of the user's face, the system comprising:

- a device for acquiring a video as described above;
- a data processing unit capable of characterizing skin alterations in the part of the face of the user acquired by the camera from the video.

Such a system is capable of using the video acquired by the camera of the acquiring device, to characterize alteration(s) to the user's skin.

The system according to the invention may have the following characteristics:

- the data processing unit is capable of:

  - identifying skin alterations in the part of the user's face acquired by the camera of the acquiring device, on the video,
  - determining at least one characteristic of each identified skin alteration, and
  - comparing the determined characteristics or some of the determined characteristics with a data base so as to characterize the skin alteration.

Another purpose of the invention is a method for characterization of alterations to a user's skin making use of a system as described above, the method including:

- an arrangement of the frame on the user's face in front of the user's eyes,
- acquisition of a video of part of the user's face by the camera, and
- characterization of the skin alteration(s) in the part of the user's face acquired by the camera, by the data processing unit from the video.

The method may comprise one or several of the following characteristics taken in isolation or in any technically possible combination:

- the characterization step includes:

  - identification of the skin alteration or some of the skin alterations in the part of the user's face acquired by the camera, on the video,
  - determination of at least one characteristic of each identified skin alteration, and
  - comparison of the determined characteristics with a data base so as to characterize the skin alteration.

- the part of the user's face acquired by the camera is at least part of a peripheral zone of one of the user's eyes.
- the acquisition step is performed over several hours, preferably continuously, and advantageously during a single day between 8:00 and 22:00.
- the method includes a prior step in which a cosmetic product is applied to the skin alteration(s) in the part of the user's face acquired by the camera.

The invention also relates to the use of a system like that described above to characterize skin alterations such as the wrinkles of a user, said characterization including at least one functional trial step.

The invention also relates to the use of a system like that described above to determine the evolution of one or more alterations in the user's skin after application of a cosmetic product on said skin alteration(s).

The invention also relates to a device according to claim 1.

According to other advantageous aspects of the invention, the device comprises one or more of the features of claims 2 to 4.

The invention also relates to a system according to claim 5.

The system can comprises the features of claim 6.

The invention also relates to a method according to claim 7.

According to other advantageous aspects of the invention, the method comprises one or more of the features of claims 8 to 10.

The invention also concerns a use of the system according to claim 11 or 12. The invention will be easier to understand after reading the following description, provided solely as an example, and with reference to the appended drawings, wherein:

- figure 1 is a schematic view of a user and a system for characterization of the user's wrinkles according to the invention;
- figure 2 is a schematic perspective view of an acquiring device of the system in figure 1.

- figure 3 is another schematic view of the device in figure 2;
- figure 4 is a schematic view of the lighting unit of the device in figure 2, and
- figure 5 is a schematic view of the operation of a variant of a lighting unit of the device in figure 2, and

For the purposes of this application, the term "video" denotes a plurality of successive images of a particular zone of interest. Such images are acquired at a predetermined frequency for a predetermined duration as a function of the application of interest.

Throughout the description, the term "transverse" is defined in relation to a straight line passing through the center of the eyes of a user. The term "longitudinal" is defined relative to a direction approximately perpendicular to the transverse direction in a horizontal plane, in other words extending between the user's face and the back of the user's head. The direction perpendicular to the longitudinal direction and to the transverse direction is called the "vertical" direction.

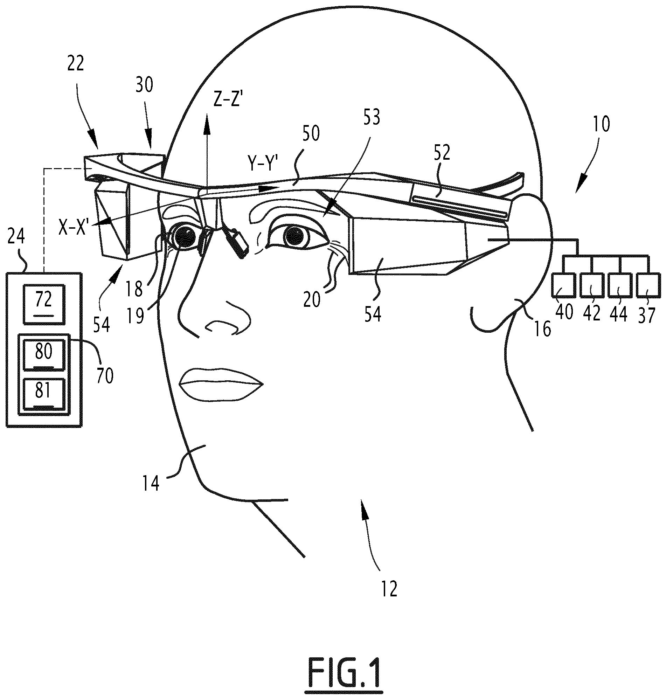

A system 10 for characterization of skin alterations of a user 12 is illustrated on figure 1.

Skin alterations 20 are permanent alterations or alterations that appear during a movement of the face 14 of the user 12.

In the following, the skin alterations are wrinkles. As a variant, the skin alterations consist of micro-relief, eye bags, pores, pimples, beauty spots, scars or any other excrescence or dimple present on the face of the user 12.

In one variant, the skin alterations 20 are complexion type alterations such as dark circles or skin discolorations.

In another variant, the skin alterations 20 include hair or keratinic fibers projecting from the skin and particularly from the eyelashes of the user 12.

The user 12 has a face 14 and ears 16. The face 14 comprises two eyes 18 each with an iris 19, and at least one wrinkle 20.

For example, the wrinkles 20 are called "mechanical" wrinkles. Such mechanical wrinkles appear as a result of repeated contractions of the muscles in the user's face. Such wrinkles are also known as "expression wrinkles". For example, mechanical wrinkles are transverse wrinkles on the user's forehead, wrinkles between the user's eyebrows called "lion wrinkles", and wrinkles at the extreme corners of the user's eyes called "crow's foot wrinkles".

In addition or as a variant, the wrinkles 20 of the user 12 are static wrinkles. Such static wrinkles are due to a general relaxation of skin tissues, to gravity and to photo-aging. For example, such static wrinkles are wrinkles that develop between the corners of the mouth and the chin called "bitterness folds" or wrinkles from the sides of the nose to the corners of the mouth called "nasogenian folds".

The wrinkle characterization system 10 comprises a video acquiring device 22 and a data processing unit 24.

The video acquiring device 22 is configured to acquire a video of the wrinkles 20 of a user 12, and particularly the wrinkles 20 located on the face 14 of the user 12. Such wrinkles may for example be crow's foot wrinkles.

In the embodiment illustrated on figures 1 to 3, the device 22 comprises a frame 30, a camera 34, at least one lighting unit 36A, an optical reflection unit 36B, a control unit 37, a battery 38 and a storage card 40. In addition, the device 22 also comprises a sensor 42 and/or a microphone 44.

The frame 30 will be placed on the face 14 of the user 12 in front of the eyes 18 of the user 12.

For example, the frame 30 may be the frame of a pair of spectacles.

The frame 30 may for example be made of aluminum. Such a material has the advantage of being lightweight which improves the comfort of the user 12 when wearing the frame 30. The expression "lightweight material" means a material with a density of less than or equal to 3 grams per cubic centimeter (g/cm$^3$).

Furthermore, such a material helps to cool the frame 30 when the camera 34 and the lighting unit 36A are in operation, without using a fan to avoid adding any nuisance noise.

As a variant, the frame 30 is made from any other lightweight and/or heat dissipating material.

The frame 30 also comprises a front frame 50 and two temples 52.

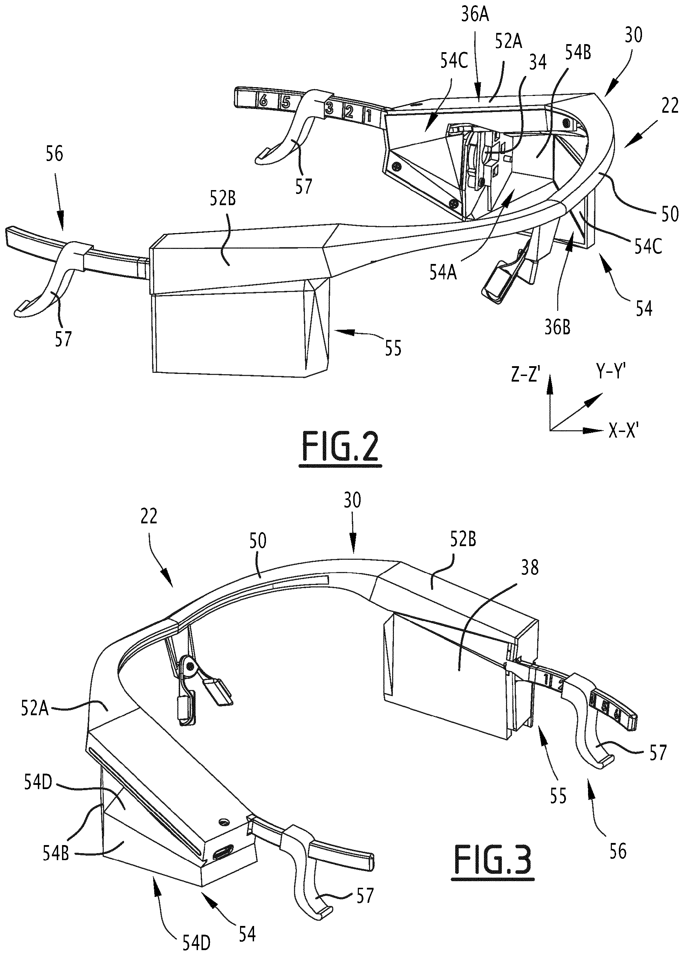

The front frame 50 extends in the longitudinal direction along a longitudinal axis X-X', in the transverse direction along a transverse axis Y-Y', and in the vertical direction along a vertical axis Z-Z'. Such longitudinal X-X', transverse Y-Y' and vertical Z-Z' axes are illustrated on figures 1 and 2.

In the example illustrated on figure 1, the front bridge 50 comprises two half-circles 53.

Each half-circle 53 is placed facing one eye 18 of the user 12 when the frame 30 is placed on the face 14 of the user 12. Each half-circle 53 will hold one lens of a pair of spectacles, such as a corrective lens.

As a variant, the front bridge 50 comprises a circle to replace each half-circle 53.

Each temple 52 projects from one of the ends of the front bridge 50 along the longitudinal axis X-X'.

Each temple 52 will rest on one ear 16 of the user 12. Each temple 52 will connect one of the half-circles 53 to one ear 16 of the user 12 when the frame 30 is placed on the face 14 of the user 12.

One of the temples 52A comprises a housing 54 that holds the camera 34, the lighting unit 36A and the optical reflection unit 36B.

As can be seen on figures 2 and 3, the housing 54 is composed of a cavity 54A delimited by two transverse walls 54B along the transverse axis Y-Y', two longitudinal walls 54C along the longitudinal axis X-X' and two vertical walls 54D along the vertical axis Z-Z'.

The other temple 52B comprises a housing 55 that holds the battery 38.

In the embodiment illustrated on figures 1 to 4, the frame 30 also comprises a system 56 to adjust the position of the frame 30 when the frame 30 is placed on the face 14 of the user 12.

The adjustment system 56 comprises two rods 57. Each rod 57 fits on a distinct temple 52 of the frame 30.

Each rod 57 can also be displaced in translation on the corresponding temple 52. This enables the rods 57 to become blocked in contact with the ears 16 of any user 12 when the frame 30 is placed in the face 14 of the user 12. Thus, the frame 30 is easily adjusted to suit all morphologies of users 12.

The camera 34 comprises an objective and a field of vision. The field of vision of a camera is defined with respect to the field angle of the camera. The field angle α of the camera is given by the following equation:

$$\alpha = 2.\arctan\left(\frac{d}{2.f}\right)$$

in which

- d is the length of an edge or the diagonal of the optical image, in other words the film or sensor of the camera,
- f is the focal distance of the objective, and
- arctan(X) is the arc tangent function of X.

The camera 34 is fixed on the frame 30 such that a part of the face 14 of the user 12 is directly or indirectly in the field of vision of the camera 34 when the frame 30 is located on the face 14 of the user 12 facing the eyes 18 of the user 12, said part of the face 14 comprising the skin alteration or at least one skin alteration 20, such as a wrinkle. An image of said part of the face 14 will be acquired by the camera 34.

An object is considered to be directly in the field of vision of the camera when the objective of the camera is oriented directly towards said object so that the camera can acquire the image of said object. An object is considered to be indirectly in the field of vision of the camera when the objective of the camera is not oriented directly towards said object, but is oriented towards a surface capable of reflecting the image of said object to the objective, for example by reflection.

Preferably, the direct or indirect field of vision of the camera 34 is centered on a part of the user's face 14 other than the irises 19 when the frame 30 is placed on the face 14 of the user 12 in front of the eyes 18 of the user 12. Thus, the irises 19 of the user are not present at the center of the images of the face 14 of the user 12 acquired by the camera 34.

In the embodiment illustrated on figures 1 to 4, the camera 34 is placed in the housing 54 of the frame 30 so as to acquire a video of part of a peripheral zone around an eye 18 of the user 12. More precisely, the peripheral zone is the contour of one eye 18 of the user 12, and the alterations are, for example, crow's foot wrinkles.

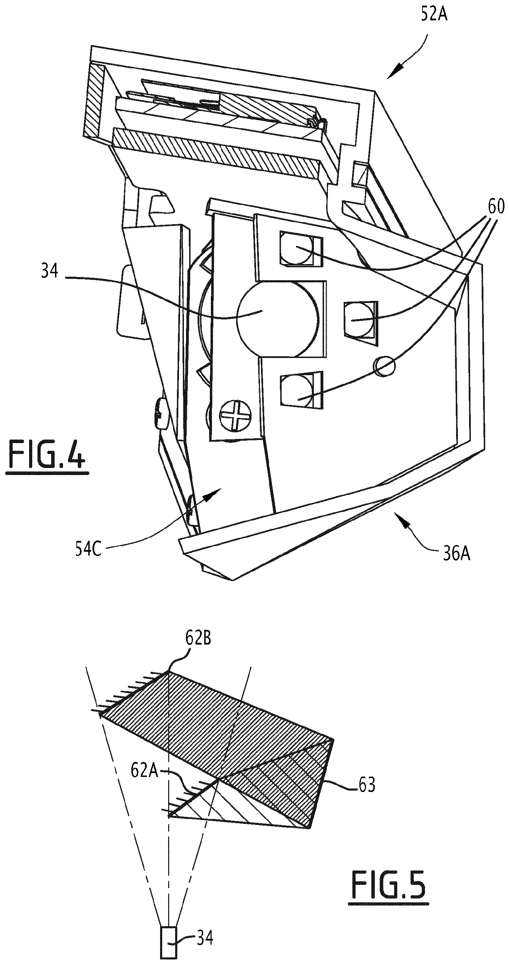

Preferably, as can be seen on figures 2 and 4, the camera 34 is fixed in the housing 54 of the temple 52A of the frame 30, for example on the longitudinal wall 54C furthest from the front bridge 50 along the longitudinal axis X-X'.

The optical axis of the camera 34 is preferably oriented towards the front bridge 50 in the horizontal plane, in other words in the plane defined by the longitudinal X-X' axis and the transverse axis Y-Y',

As a variant, the camera 34 is placed on another wall of the cavity 54A such that the field of vision of the camera 34 corresponds to the part of interest of the face 14 of the user 12.

The lighting unit 36A is fixed to the frame 30, and more precisely, to one of the temples 52 of the frame 30. The lighting unit 36A is capable of lighting the part of the face 14 directly or indirectly in the field of vision of the camera 34 and that will be acquired by the camera 34.

In the embodiment illustrated on figures 1 to 4, the lighting unit 36A is placed in the housing 54 of the temple 52A.

In the embodiment illustrated on figures 1 to 4, the lighting unit 36A comprises at least one light emitting diode 60.

In the example illustrated on figure 4, the lighting unit 36A comprises three light emitting diodes 60.

Preferably and as can be seen on figures 2 and 4, the light emitting diodes 60 are located on the same wall 54C as the camera 34. This provides sufficient illumination for the images acquired by the camera 34.

Advantageously, the light emitting diodes 60 are distributed all around the camera 34. This enables a uniform illumination of images acquired by the camera 34.

Preferably, each light emitting diode 60 can be activated either individually and/or automatically. This makes it possible to adjust the lighting as a function of ambient lighting conditions, and particularly to compensate for non-optimal ambient lighting conditions.

Optionally, the lighting unit 36A comprises a cache to protect the eyes 18 of the user 12 when the diodes 60 are in use.

Optionally, the diodes 60 are placed on a wheel called a "LED wheel" such that, when the wheel is turned, the angle of incidence of light emitted by the diodes 60 changes. This makes it possible to find more complete information about the visibility of skin alterations 20, or even information in three dimensions.

When the camera 34 comprises an objective not oriented directly towards the part of the face 34 to be acquired by the camera 34, the optical reflection unit 36B is oriented such that at least some of the light waves emitted by the part of the face 34 for which the image is to be acquired are reflected to the objective of the camera 34.

For example, the optical reflection unit 36B comprises a mirror 62 oriented so as to reflect light waves emitted by the part of the face 34 of which the image is to be acquired and sent to the objective of the camera 34. Thus, the mirror 62 is in the field of vision of the camera 34. This enables the camera 34 to acquire images of parts of the face 14 of the user 12 reflected by the mirror and that are not in the direct field of vision of the camera 34. The parts of the face 14 are then indirectly in the field of vision through the mirror.

In addition, the lighting unit 36A is not oriented directly towards the part of the face 34 that will be acquired by the camera 34 and the optical reflection unit 36B is oriented such that at least some of the light waves emitted by the lighting unit 36A are reflected to said part of the face 14. The lighting unit 36A and the objective of the camera 34 are then preferably located close to each other, particularly on the same temple 52.

Thus, the mirror 62 is placed in the housing 54 such that at least some of the light waves emitted by the light emitting diodes 60 of the optical reflection unit 36B are reflected on the mirror 62 towards the part of the face 14 of the user 12 acquired by the camera 34. Thus, the part of the face 14 is illuminated by light waves from the light emitting diodes 60.

As a variant, the device 22 comprises two distinct optical reflection units: the first being oriented such that at least some of the light waves emitted by the part of the face 34 of which the image is to be acquired are reflected to the objective of the camera 34, and the second being oriented such that at least some of the light waves emitted by the lighting unit 36A is reflected to said part of the face 14.

As can be seen on figure 2, the mirror 62 is placed on the longitudinal wall 54C on the opposite side of the longitudinal wall 54C on which the diodes 60 are placed, along the longitudinal axis X-X'.

The mirror 62 is for example a plane mirror.

Preferably, the light emitting diodes 60 are positioned at a distance equal to or less than 10 centimeters (cm) from the mirror 62. This results in a compact device 22. Furthermore, the presence of light emitting diodes 60 close to the mirror 62 prevents condensation on the mirror 62, for example when moving from one location to another at a different temperature.

Advantageously, the lighting unit 36A is arranged such that the direction of illumination of light waves reflected on the mirror 62 is not very different from the direction of observation of the user 12. This can optimize lighting of skin alterations 20 of the part of the face 14 of the user 12. Furthermore, such an arrangement can obstruct part of the external light and thus avoid disturbing the acquisition of videos due to the presence of an environment such as sunlight in which light is too intense.

According to the invention, the optical reflection device 36B comprises two mirrors 62A, 62B arranged relative to each other and to the camera 34 such that the video acquired by the camera 34 is a stereoscopic video, in other words a video in three dimensions.

For example, as illustrated on figure 5, the mirrors 62A and 62B are located one behind the other and are offset such that a part of the field of vision of the camera 34 is derived from reflections of an image of an object 63 on one of the mirrors 62A and the other part of the field of vision of the camera 34 is derived from reflections of the image of the object 63 on the other mirror 62B.

In another example, the mirrors 62A and 62B are capable of pivoting. Initially, the mirrors 62A, 62B are located one behind the other such that mirror 62B is masked by mirror 62A in the field of vision of the camera 34. The camera 34 is then capable of acquiring a first image corresponding to reflections on the mirror 62A.. The mirror 62A can then be pivoted such that the field of vision of the camera 34 corresponds to the mirror 62B and therefore to reflections on the mirror 62B. The camera 34 is then capable of acquiring a second image corresponding to reflections on mirror 62B. The first image and the second image can be merged to obtain a stereoscopic image.

As another variant, diffusing panels are arranged all around the mirror(s) 62. For example, the diffusing panels may be made of frosted glass. Such diffusing panels can be used to create illumination with diffuse light.

The control unit 37 is capable of communicating with the camera 34 and the lighting unit 36A so as to control activation or deactivation of the camera 34 and also of the lighting unit 36A.

The control unit 37 is also capable of extracting information memorized on the storage card 40 and sending it to the data processing unit 24.

For example, the control unit 37 comprises a processor, a memory and a user interface.

The control unit 37 can preferably be remote controlled by an electronic device of the user 12. Such a remote control may for example by made by Wi-Fi or Bluetooth. The electronic device may for example by a smart phone or a computer on which a dedicated application is installed. For example, the electronic device can be used to display an image acquired by the camera 34, to make adjustments to the camera 34 or to the lighting unit 36A, or to control the camera 34, in real time.

For example, the control unit 37 may be positioned in the housing 54 on the temple 52A.

The battery 38 is configured to supply power to the camera 34, the lighting unit 36A and the control unit 37. Such a battery 38 thus assures energy independence of the acquiring device 22, that then does not have to be connected to a domestic energy source.

The battery 38 is fixed to the frame 30, and more precisely to one of the temples 52 of the frame 30.

In the embodiment illustrated on figures 1 to 4, the battery 38 is fixed in the housing 55 of the temple 52B. The position of the battery 38 on the temple 52B and not on the temple 52A that already supports the camera 34 and the lighting unit 36A distributes the weight of the different elements on the two temples 52A, 52B of the frame 30. This is more comfortable for the user 12.

Preferably, the battery 38 is fixed in the housing 55 by clips. This makes it easy to replace the battery 38 when said battery 38 is discharged.

Furthermore, the acquiring device 22 also includes an additional battery 70 placed on the same temple 52A as the camera 34 and close to said camera 34. Such an additional battery 70 can provide assurance of power supply to the camera 34 and the lighting unit 36A even when the battery 38 is being replaced.

The storage card 40 is connected to the camera 34 so as to store videos acquired by the camera 34. Such a storage card 40 can thus extend the storage capacity of the camera 34.

The storage card 40 is fixed to the frame 30. Preferably, the storage card 40 is placed in the housing 54 of the temple 52A so as to be close to the camera 34.

The storage card 40 may for example be a micro SD card. Such a storage card 40 can be used to store videos at high speed.

The sensors 42 are capable of providing information about the environment of the user 12. Such sensors 42 are capable of providing information about the environment of the user 12. Such information can then be correlated with the characteristics of skin alterations 20 on the part of the face 14 of the user 12 acquired by the camera 34.

For example, such information may be the geographic position of the user 12 when the sensor 42 is a GPS (*Global Positioning System;* the temperature of the environment of the user 12 when the sensor 42 is a temperature sensor; the humidity of the environment of the user 12 when the sensor 42 is a hygrometer; or the sunlight in the environment of the user 12 when the sensor 42 is a sunlight sensor.

Furthermore, the sensors 42 are cameras oriented towards the environment of the user 12, for example towards persons in contact with the user 12. This makes it possible to make a correlation between the movements of the wrinkles 20 of the user 12 and interactions between the user 12 and his or her environment.

The microphone 44 may for example by fixed to the frame 30, for example to one of the temples 52 of the frame 30.

The microphone 44 is capable, for example, of picking up sounds made by the user 12. As a result, a correlation can be made between movements of the wrinkles 20 of the user 12 and sounds pronounced by the user 12.

Furthermore and optionally, at least one item among the camera 34, the lighting unit 36A, the optical reflection unit 36B, the control unit 37, the storage card 40, the sensor 42 and the microphone 44 is fixed by clips onto the frame 30 to make it easy to modulate the device 22.

The data processing unit 24 is positioned at a distance from the acquiring device 22 and is connected to the control unit 37 of the device 22. The connection is preferably a wireless connection such as a Wi-Fi or a Bluetooth connection.

As a variant, the data processing unit 24 is integrated into the device 22 and is fixed to the frame 30 of the device 22.

The data processing unit 24 is capable of using the video acquired by the device 22 to characterize the skin alteration(s) 20 to the part of the face 14 of the user 12 acquired by the camera 34.

The data processing unit 24 comprises a memory 70 and a processor 72.

The memory 70 contains a stored application 80 that, when it is run by the processor 72, is capable of characterizing skin alterations 20 in the part of the face 14 of the user 12 acquired by the camera 34.

The memory 70 also comprises a database 81 in which information about predetermined skin alterations is stored.

The application 80 is capable of identifying skin alterations 20 in the part of the face 14 of the user 12 acquired by the camera 34 of the acquiring device 22, on the video,

Such identification may for example be made by the detection of gradients in each image of the acquired video, to identify skin alterations corresponding to the determined gradients.

The application 80 is also capable of determining one or more of the characteristics of each identified skin alteration 20. The characteristics of each skin alteration are, for example, the size, color, depth or shape of the skin alteration.

The application 80 is also capable of comparing the determined characteristic(s) with the data base 81 so as to characterize the skin alteration 20.

When the skin alteration 20 corresponds to a skin alteration in the database 81, the skin alteration is identified as being of the same type as the skin alteration in the database 81.

Advantageously, the identified skin alteration 20 is added to the database 81.

A method of using the characterization system 10 will now be described.

Initially, the frame 30 of the acquiring device 22 will be placed on the face 14 of the user 12 in front of the eyes 18 of the user 12.

The control unit 37 controls activation of the camera 34 and the lighting unit 36A.

The lighting unit 36A illuminates part of the face 14 of the user 12, possibly taking account of the lighting of the environment of the user 12.

In parallel, the camera 34 acquires a video of part of the face 14 of the user 12. The acquired video is memorized on the storage card 40 in real time.

When the acquiring device 22 comprises sensors 42 or a microphone 44, such elements can provide complementary information about the environment of the user 12 or sounds emitted by the user 12.

The control unit 37 then sends the video acquired by the camera 34 and any information measured by the sensors 42 and/or the microphone 44, to the data processing unit 24.

The processor 72 of the data processing unit 24 working in interaction with the characterization application 80 identifies the skin alteration(s) 20 of the part of the face 14 of the user 12 acquired by the camera 34 on the video, and then determines the characteristics of each identified skin alteration 20.

The processor 72 working in interaction with the characterization application 80 compares the determined characteristic(s) with the data base 81 so as to characterize the skin alteration 20.

When the skin alteration 20 corresponds to a skin alteration in the database 81, the skin alteration 20 is identified as being of the same type as the skin alteration in the database 81.

Advantageously, the identified skin alteration 20 is added to the database 81.

Thus, the system 10 can be used to characterize skin alterations such as wrinkles and therefore subjacently the effects of cosmetic products, such as "anti-wrinkle" cosmetic products.

The acquiring device 22 also has the advantage of being entirely wireless and therefore can be used without hindrance to the user 12.

Therefore the acquiring device 22 can be used for the acquisition of images of skin alterations in real time, dynamically and under everyday conditions.

In the embodiments illustrated on figures 1 to 4, the camera 34 and the lighting unit 36A are arranged on the frame 30 so as to acquire a video of the contour of the eye of the user 12 and particular the crow's foot wrinkles of the user 12. However, an expert in the subject will understand that that the camera 34 and the lighting unit 36A can be positioned at other locations on the face 14 of the user 12 so that videos of other types of skin alteration of the user 12 can be acquired. For example, the camera 34 and the lighting unit 36A could be positioned on the frame 30 so as to acquire wrinkles on the forehead of the user 12.

An expert in the subject will also understand that other cameras 34, lighting units 36A and optical reflection units 36B can be added to the acquiring device 22 so that videos of other parts of the face 14 of the user 12 can be acquired in parallel, including other types of skin alterations (wrinkles under the eyes, wrinkles of the forehead, eye bags, dark rings).

In addition, the system 10 can be used to characterize skin alterations of a user 12 when the user 12 performs at least one functional test. For example, a functional test is the reproduction of a predefined sound by the user 12 or reproduction of predefined movements of the face, such as frowns. Patent FR 3 014 675 gives examples of such functional tests.

## Claims

1. Device (22) for acquiring a video of skin alterations (20) of a user (12), the user (12) having a face (14) comprising at least one skin alteration (20) and two eyes (18), the skin alteration(s) (20) being permanent alterations or alterations that appear during a movement of the face (14) of the user (12), the device (22) comprising:

   - a frame (30) intended to be placed on the face (14) of the user (12) in front of the eyes (18) of the user (12), and
   - a camera (34) fixed on the frame (30), the camera (34) comprising a field of vision, the camera (34) being fixed on the frame (30) such that a part of the face (14) of the user (12) is indirectly in the field of vision of the camera (34) when the frame (30) is placed on the face (14) of the user (12) in front of the eyes (18) of the user (12), said part of the face (14) comprising the or at least one skin alteration (20), and an image of said part of the face (14) being intended to be acquired by the camera (34), the camera (34) comprising an objective not oriented directly towards the part of the face (34) to be acquired by the camera (34),
   - a first optical reflection unit (36B) oriented such that at least some of the light waves emitted by the part of the face (14) for which the image is to be acquired are reflected to the objective of the camera (34), the first optical reflection unit (36B) comprising two mirrors (62A, 62B) positioned such that the video acquired by the camera (34) is a stereoscopic video
   - at least one lighting unit (36A) fixed to the frame (30), the lighting unit (36A) being capable of lighting at least part of the face (14) directly or indirectly in the field of vision of the camera (34) and that will be acquired by the camera (34), the lighting unit (36A) being not oriented directly towards the part of the face (34) intended to be acquired by the camera (34), and
   - a second optical reflection unit oriented such that at least some of the light waves emitted by the lighting unit (36A) are reflected to said part of the face (14).

2. Device (22) according to claim 1, in which when the camera (34) is fixed on the frame (30) such that when the frame (30) is placed on the face (14) of the user (12) in front of the eyes (18) of the user (12), the part of the face (14) that is directly or indirectly in the field of vision of the camera (34) is part of a peripheral zone of an eye (18) of the user (12).

**3.** Device (22) according to claim 1 or 2, in which each eye (18) of the user (12) comprises an iris (18), the field of vision of the camera (34) being directly or indirectly centered on a part of the face (14) of the user except for the irises (19) when the frame (30) is placed on the face (14) of the user (12) in front of the eyes (18) of the user (12).

**4.** Device (22) according to any one of claims 1 to 3, in which the frame (30) comprises two circles or half-circles (53) intended to be positioned in front of the eyes (18) of the user (12) and temples (52) each connecting a circle or half-circle (53) to an ear (16) of the user (12), each temple (52) being intended to rest on an ear (16) of the user (12), the camera (34) being fixed to one of the temples (52).

**5.** System (10) for characterization of skin alterations (20) of a user (12), the user (12) having a face (14) comprising at least one skin alteration (20) and two eyes (18), the skin alteration(s) (20) being permanent alterations or alterations that appear during a movement of the face (14) of the user (12), the system (10) comprising:

- a device (22) for acquiring a video according to any one of claims 1 to 4, and
- a data processing unit (24) capable of characterizing the skin alternation(s) (20) in the part of the face (14) of the user (12) acquired by the camera (34) from the video.

**6.** System (10) according to claim 5, in which the data processing unit (24) is capable of:

- identifying skin alterations (20) in the part of the face (14) of the user (12) acquired by the camera (34) of the acquiring device (22), on the video,
- determining at least one characteristic of each identified skin alteration (20), and
- comparing the determined characteristics or some of the determined characteristics with a data base so as to characterize the skin alteration (20).

**7.** Method for identifying skin alterations (20) of a user (12) making use of a system according to claim 5 or 6, the method including:

- an arrangement of the frame (30) on the face (14) of the user (12) in front of the eyes (18) of the user (12),
- acquisition of a video of part of the face (14) of the user (12) by the camera (34), and
- characterization of the skin alteration(s) (20) in the part of the face (14) of the user (12) acquired by the camera (34), by the data processing unit (24), from the video.

**8.** Characterization method according to claim 7, in which the characterization step comprises:

- identification of the skin alteration or some of the skin alterations (20) in the part of the face (14) of the user (12) acquired by the camera (34), on the video,
- determination of at least one characteristic of each identified skin alteration (20), and
- comparison of the determined characteristics with a data base so as to characterize the skin alteration (20).

**9.** Characterization method according to claim 7 or 8, in which the part of the face (14) of the user (12) acquired by the camera (34) is at least part of a peripheral zone an eye (18) of the user (12).

**10.** Characterization method according to any one of claims 7 to 9, in which the acquisition step is performed over several hours, preferably continuously, and advantageously during a single day between 8:00 and 22:00.

**11.** Use of a system (10) according to claim 5 or 6, to characterize skin alterations such as the wrinkles (20) of a user (12), said characterization including at least one functional trial step.

**12.** Use of a system (10) according to claim 5 or 6 to determine the evolution of one or more alterations (20) in the skin of the user (12) after application of a cosmetic product on said skin alteration(s) (20).

**Patentansprüche**

**1.** Vorrichtung (22) zum Aufnehmen eines Videos von Hautveränderungen (20) eines Benutzers (12), wobei der Benutzer (12) ein Gesicht (14) aufweist, umfassend mindestens eine Hautveränderung (20) und zwei Augen (18), wobei die Hautveränderung(en) (20) permanente Veränderungen oder Veränderungen sind, die während einer Bewegung des Gesichts (14) des Benutzers (12) erscheinen, die Vorrichtung (22) umfassend:

- ein Gestell (30), das dazu bestimmt ist, auf dem Gesicht (14) des Benutzers (12) vor den Augen (18) des Benutzers (12) platziert zu werden, und
- eine Kamera (34), die an dem Gestell (30) befestigt ist, die Kamera (34) umfassend ein Sichtfeld, wobei die Kamera (34) an dem Gestell (30) befestigt ist, sodass ein Teil des Gesichts (14) des Benutzers (12) indirekt in dem Sichtfeld der Kamera (34) ist, wenn das Gestell (30) auf dem Gesicht (14) des Benutzers (12) vor den Augen (18) des Benutzers (12) platziert ist, der Teil des Gesichts (14) umfassend die oder mindestens eine Hautveränderung (20), und wobei beabsichtigt ist, dass ein Bild des Teils des Gesichts (14) von der Kamera (34) aufgenommen wird, die Kamera (34) umfassend ein Objektiv, das nicht direkt auf den Teil des Gesichts (34) ausgerichtet ist, der von der Kamera (34) aufgenommen werden soll,

- eine erste optische Reflexionseinheit (36B), die ausgerichtet ist, sodass mindestens ein Teil der Lichtwellen, die von dem Teil des Gesichts (14), von dem das Bild aufgenommen werden soll, emittiert werden, zu dem Objektiv der Kamera (34) reflektiert wird, die erste optische Reflexionseinheit (36B) umfassend zwei Spiegel (62A, 62B), die positioniert sind, sodass das von der Kamera (34) aufgenommene Video ein stereoskopisches Video ist

- mindestens eine Beleuchtungseinheit (36A), die an dem Gestell (30) befestigt ist, wobei die Beleuchtungseinheit (36A) in der Lage ist, mindestens einen Teil des Gesichts (14) direkt oder indirekt in dem Blickfeld der Kamera (34) und der von der Kamera (34) aufgenommen werden soll, zu beleuchten, wobei die Beleuchtungseinheit (36A) nicht direkt auf den Teil des Gesichts (34) ausgerichtet ist, der von der Kamera (34) aufgenommen werden soll, und

- eine zweite optische Reflexionseinheit, die ausgerichtet ist, sodass mindestens ein Teil der von der Beleuchtungseinheit (36A) emittierten Lichtwellen auf den Teil des Gesichts (14) reflektiert wird.

2. Vorrichtung (22) nach Anspruch 1, wobei, wenn die Kamera (34) an dem Gestell (30) befestigt ist, sodass, wenn das Gestell (30) auf dem Gesicht (14) des Benutzers (12) vor den Augen (18) des Benutzers (12) platziert ist, der Teil des Gesichts (14), der direkt oder indirekt in dem Blickfeld der Kamera (34) ist, Teil eines peripheren Bereichs eines Auges (18) des Benutzers (12) ist.

3. Vorrichtung (22) nach Anspruch 1 oder 2, wobei jedes Auge (18) des Benutzers (12) eine Iris (18) umfasst, wobei das Sichtfeld der Kamera (34) direkt oder indirekt auf einen Teil des Gesichts (14) des Benutzers mit Ausnahme der Iris (19) zentriert ist, wenn das Gestell (30) auf dem Gesicht (14) des Benutzers (12) vor den Augen (18) des Benutzers (12) platziert ist.

4. Vorrichtung (22) nach einem der Ansprüche 1 bis 3, das Gestell (30) umfassend zwei Kreise oder Halbkreise (53), die dazu bestimmt sind, vor den Augen (18) des Benutzers (12) positioniert zu werden, und Bügel (52), die jeweils einen Kreis oder Halbkreis (53) mit einem Ohr (16) des Benutzers (12) verbinden, wobei jeder Bügel (52) dazu bestimmt ist, auf einem Ohr (16) des Benutzers (12) aufzuliegen, wobei die Kamera (34) an einem der Bügel (52) befestigt ist.

5. System (10) zur Charakterisierung von Hautveränderungen (20) eines Benutzers (12), wobei der Benutzer (12) ein Gesicht (14) aufweist, umfassend mindestens eine Hautveränderung (20) und zwei Augen (18), wobei die Hautveränderung(en) (20) permanente Veränderungen oder Veränderungen sind, die während einer Bewegung des Gesichts (14) des Benutzers (12) erscheinen, das System (10) umfassend:

- eine Vorrichtung (22) zum Aufnehmen eines Videos nach einem der Ansprüche 1 bis 4, und
- eine Datenverarbeitungseinheit (24), die in der Lage ist, die Hautveränderung(en) (20) in dem Teil des Gesichts (14) des Benutzers (12) zu charakterisieren, der von der Kamera (34) aus dem Video aufgenommen wird.

6. System (10) nach Anspruch 5, wobei die Datenverarbeitungseinheit (24) zu Folgendem in der Lage ist:

- Identifizieren von Hautveränderungen (20) in dem Teil des Gesichts (14) des Benutzers (12), der von der Kamera (34) der Aufnahmevorrichtung (22) aufgenommen wird, auf dem Video,
- Bestimmen mindestens einer Charakteristik jeder identifizierten Hautveränderung (20), und
- Vergleichen der bestimmten Charakteristiken oder einiger der bestimmten Charakteristiken mit einer Datenbank, um die Hautveränderung (20) zu charakterisieren.

7. Verfahren zum Identifizieren von Hautveränderungen (20) eines Benutzers (12) unter Verwendung eines Systems nach Anspruch 5 oder 6, wobei das Verfahren Folgendes beinhaltet:

- eine Anordnung des Gestells (30) auf dem Gesicht (14) des Benutzers (12) vor den Augen (18) des Benutzers (12),
- Aufnehmen eines Videos von einem Teil des Gesichts (14) des Benutzers (12) durch die Kamera (34), und
- Charakterisieren der Hautveränderung(en) (20) in dem Teil des Gesichts (14) des Benutzers (12), der von der Kamera (34) aufgenommen wird, durch die Datenverarbeitungseinheit (24) aus dem Video.

8. Charakterisierungsverfahren nach Anspruch 7, wobei der Charakterisierungsschritt Folgendes umfasst:

- Identifizieren der Hautveränderung oder einiger der Hautveränderungen (20) in dem von der Kamera (34) aufgenommenen Teil des Gesichts (14) des Benutzers (12) auf dem Video,
- Bestimmen mindestens einer Charakteristik jeder identifizierten Hautveränderung (20), und
- Vergleichen der bestimmten Charakteristiken mit einer Datenbank, um die Hautveränderung (20) zu charakterisieren.

9. Charakterisierungsverfahren nach Anspruch 7 oder 8, wobei der von der Kamera (34) aufgenommene Teil des Gesichts (14) des Benutzers (12) mindestens ein Teil eines peripheren Bereichs eines Auges (18) des Benutzers (12) ist.

10. Charakterisierungsverfahren nach einem der Ansprüche 7 bis 9, wobei der Aufnahmeschritt über mehrere Stunden, vorzugsweise kontinuierlich, und vorteilhafterweise während eines einzigen Tages zwischen 8:00 und 22:00 Uhr ausgeführt wird.

11. Verwendung eines Systems (10) nach Anspruch 5 oder 6, um Hautveränderungen, wie beispielsweise die Falten (20) eines Benutzers (12) zu charakterisieren, wobei die Charakterisierung mindestens einen funktionellen Testschritt beinhaltet.

12. Verwendung eines Systems (10) nach Anspruch 5 oder 6, um die Entwicklung einer oder mehrerer Veränderungen (20) in der Haut des Benutzers (12) nach Auftragen eines kosmetischen Mittels auf die Hautveränderung(en) (20) zu bestimmen.

**Revendications**

1. Dispositif (22) pour l'acquisition d'une vidéo d'altérations de la peau (20) d'un utilisateur (12), l'utilisateur (12) ayant un visage (14) comprenant au moins une altération de la peau (20) et deux yeux (18), la ou les altérations de la peau (20) étant des altérations permanentes ou apparaissant lors d'un mouvement du visage (14) de l'utilisateur (12), le dispositif (22) comprenant :

- une monture (30) destinée à être placée sur le visage (14) de l'utilisateur (12) devant les yeux (18) de l'utilisateur (12), et
- une caméra (34) fixée sur la monture (30), la caméra (34) comprenant un champ de vision, la caméra (34) étant fixée sur la monture (30) de telle sorte qu'une partie du visage (14) de l'utilisateur (12) se trouve indirectement dans le champ de vision de la caméra (34) lorsque la monture (30) est placée sur le visage (14) de l'utilisateur (12) devant les yeux (18) de l'utilisateur (12), ladite partie du visage (14) comprenant la ou au moins une altération de la peau (20), et une image de ladite partie du visage (14) étant destinée à être acquise par la caméra (34), la caméra (34) comprenant un objectif non orienté directement vers la partie du visage (34) destinée à être acquise par la caméra (34),
- une première unité de réflexion optique (36B) orientée de manière à ce qu'au moins certaines des ondes lumineuses émises par la partie du visage (14) dont l'image doit être acquise soit réfléchie vers l'objectif de la caméra (34), la première unité de réflexion optique (36B) comprenant deux miroirs (62A, 62B) positionnés de manière à ce que la vidéo acquise par la caméra (34) soit une vidéo stéréoscopique
- au moins une unité d'éclairage (36A) fixée à la monture (30), l'unité d'éclairage (36A) étant capable d'éclairer au moins une partie du visage (14) directement ou indirectement dans le champ de vision de la caméra (34) et qui sera acquise par la caméra (34), l'unité d'éclairage (36A) n'étant pas orientée directement vers la partie du visage (34) destinée à être acquise par la caméra (34), et
- une deuxième unité de réflexion optique orientée de manière à ce qu'au moins certaines des ondes lumineuses émises par l'unité d'éclairage (36A) soit réfléchie sur ladite partie du visage (14).

2. Dispositif (22) selon la revendication 1, dans lequel lorsque la caméra (34) est fixée sur la monture (30) de telle sorte que lorsque la monture (30) est placée sur le visage (14) de l'utilisateur (12) devant les yeux (18) de l'utilisateur (12), la partie du visage (14) qui est directement ou indirectement dans le champ de vision de la caméra (34) fait partie d'une zone périphérique d'un oeil (18) de l'utilisateur (12).

3. Dispositif (22) selon la revendication 1 ou 2, dans lequel chaque oeil (18) de l'utilisateur (12) comprend un iris (18), le champ de vision de la caméra (34) étant directement ou indirectement centré sur une partie du visage (14) de l'utilisateur à l'exception des iris (19) lorsque la monture (30) est placée sur le visage (14) de l'utilisateur (12) devant les yeux (18) de l'utilisateur (12).

4. Dispositif (22) selon l'une quelconque des revendications 1 à 3, dans lequel la monture (30) comprend deux cercles ou demi-cercles (53) destinés à être positionnés devant les yeux (18) de l'utilisateur (12) et des branches (52) reliant chacune un cercle ou demi-cercle (53) à une oreille (16) de l'utilisateur (12), chaque branche (52) étant destinée à reposer sur une oreille (16) de l'utilisateur (12), la caméra (34) étant fixée à l'une des branches (52).

5. Système (10) de caractérisation des altérations de la peau (20) d'un utilisateur (12), l'utilisateur (12) ayant un visage (14) comprenant au moins une altération de la peau (20) et deux yeux (18), la ou les altérations de la peau (20) étant des altérations permanentes ou des altérations apparaissant lors d'un mouvement du visage (14) de l'utilisateur (12), le système (10) comprenant :

    - un dispositif (22) pour acquérir une vidéo selon l'une quelconque des revendications 1 à 4, et
    - une unité de traitement de données (24) capable de caractériser la ou les alternances de la peau (20) de la partie du visage (14) de l'utilisateur (12) acquises par la caméra (34) à partir de la vidéo.

6. Système (10) selon la revendication 5, dans lequel l'unité de traitement de données (24) est capable de :

    - identifier les altérations de la peau (20) dans la partie du visage (14) de l'utilisateur (12) acquise par la caméra (34) du dispositif d'acquisition (22), sur la vidéo,
    - déterminer au moins une caractéristique de chaque altération de la peau (20) identifiée, et
    - comparer les caractéristiques déterminées ou certaines des caractéristiques déterminées avec une base de données afin de caractériser l'altération de la peau (20).

7. Procédé d'identification des altérations de la peau (20) d'un utilisateur (12) utilisant un système selon la revendication 5 ou 6, le procédé comportant :

    - une disposition de la monture (30) sur le visage (14) de l'utilisateur (12) devant les yeux (18) de l'utilisateur (12),
    - l'acquisition d'une vidéo d'une partie du visage (14) de l'utilisateur (12) par la caméra (34), et
    - la caractérisation de la ou des altérations de la peau (20) dans la partie du visage (14) de l'utilisateur (12) acquise par la caméra (34), par l'unité de traitement de données (24), à partir de la vidéo.

8. Procédé de caractérisation selon la revendication 7, dans lequel l'étape de caractérisation comprend :

    - l'identification de l'altération de la peau ou de certaines des altérations de la peau (20) dans la partie du visage (14) de l'utilisateur (12) acquise par la caméra (34), sur la vidéo,
    - la détermination d'au moins une caractéristique de chaque altération de la peau (20) identifiée, et
    - la comparaison des caractéristiques déterminées avec une base de données afin de caractériser l'altération de la peau (20).

9. Procédé de caractérisation selon la revendication 7 ou 8, dans lequel la partie du visage (14) de l'utilisateur (12) acquise par la caméra (34) est au moins une partie d'une zone périphérique d'un oeil (18) de l'utilisateur (12).

10. Procédé de caractérisation selon l'une quelconque des revendications 7 à 9, dans lequel l'étape d'acquisition est réalisée sur plusieurs heures, de préférence en continu, et avantageusement au cours d'une seule journée entre 8h00 et 22h00.

11. Utilisation d'un système (10) selon la revendication 5 ou 6, pour caractériser les altérations de la peau telles que les rides (20) d'un utilisateur (12), ladite caractérisation comportant au moins une étape d'essai fonctionnel.

12. Utilisation d'un système (10) selon la revendication 5 ou 6 pour déterminer l'évolution d'une ou plusieurs altérations (20) de la peau de l'utilisateur (12) après application d'un produit cosmétique sur ladite ou lesdites altérations (20) de la peau.

## FIG.1

## FIG.2

## FIG.3

FIG.4

FIG.5

## REFERENCES CITED IN THE DESCRIPTION

### Patent documents cited in the description

- FR 2930826 A1
- US 6433760 B1
- US 2015316771 A1
- FR 3014675 A
- FR 3014675

### Non-patent literature cited in the description

- Eye Blink Detection for Smart Glasses. **LE HOANG et al.** 2013 IEEE INTERNATIONAL SYMPOSIUM ON MULTIMEDIA. IEEE, 09 December 2013, 305-308